(19) Europäisches Patentamt
European Patent Office
Office européen des brevets

(11) Veröffentlichungsnummer: **0 372 262 A1**

# (12) EUROPÄISCHE PATENTANMELDUNG

(21) Anmeldenummer: **89120982.7**

(51) Int. Cl.5: **C07C 17/38, C07C 25/02**

(22) Anmeldetag: **11.11.89**

(30) Priorität: **24.11.88 DE 3839571**

(43) Veröffentlichungstag der Anmeldung:
**13.06.90 Patentblatt 90/24**

(84) Benannte Vertragsstaaten:
**BE CH DE FR GB IT LI**

(71) Anmelder: **BAYER AG**

**D-5090 Leverkusen 1 Bayerwerk(DE)**

(72) Erfinder: **Röhlk, Kai, Dr.**
**Odenthaler Markweg 36**
**D-5060 Bergisch Gladbach 2(DE)**

## (54) Verbessertes Verfahren zur destillativen Aufarbeitung von Gemischen, die seitenkettenchlorierte Alkylaromaten enthalten.

(57) Gemische, die seitenkettenchlorierte Alkylaromaten enthalten, werden aufgearbeitet, indem man sie vor der Destillation bei erhöhter Temperatur mit einem inerten Gas ausbläst und die Destillation in Gegenwart von Aminen und/oder chlorierten Aminen durchführt.



Xerox Copy Centre

## Verbessertes Verfahren zur destillativen Aufarbeitung von Gemischen, die seitenkettenchlorierte Alkylaromaten enthalten

Die vorliegende Erfindung betrifft eine verbesserte destillative Aufarbeitung von Gemischen, die seitenkettenchlorierte Alkylaromaten enthaltend. Bei der erfindungsgemäßen Aufarbeitung werden mit speziellen Maßnahmen Nebenreaktionen während der Destillation weitgehend eliminiert.

Seitenkettenchlorierte Alkylaromaten, z.B. Benzylchloride, Benzalchloride und Benzotrichloride, und deren Verseifungsprodukte, z.B. Benzylalkohole, Benzaldehyde, Benzoesäuren und Benzoylchloride, sind wichtige Zwischenprodukte, die in großen Mengen produziert und beispielsweise zur Herstellung von Pflanzenschutzmitteln, Pharmazeutika, Farb- und Riechstoffen verwendet werden (siehe Kirk-Othmer, Encyclopedia of Chemical Technology, 2nd Ed., Vol. 5, S. 281-289 (1964)).

Seitenkettenchlorierte Alkylaromaten werden meistens durch eine Seitenkettenchlorierung der entsprechenden Alkylaromaten hergestellt. Dabei wird bei erhöhter Temperatur und gegebenenfalls unter Bestrahlung gearbeitet, wodurch einzelne seitenkettenchlorierte Alkylaromaten stets mit nur geringer Selektivität erhalten werden und von anderen seitenkettenchlorierten Alkylaromaten und/oder Ausgangs- und/oder Nebenprodukten abgetrennt werden müssen. Die nach der Chlorierung vorliegenden Reaktionsgemische enthalten stets mehrere Komponenten. Chloriert man auf diese Art beispielsweise o-Chlortoluol, so kann man die Chlorierung beispielsweise bis zu folgenden Stufen durchführen:

Stufe I: Bis noch keine nennenswerten Mengen o-Chlorbenzalchlorid entstehen. Das Chlorierungsprodukt enthält dann im wesentlichen unumgesetztes o-Chlortoluol und o-Chlorbenzylchlorid.

Stufe II: Bis die maximale Menge o-Chlorbenzylchlorid entstanden ist. Das Chlorierungsprodukt enthält dann im wesentlichen o-Chlorbenzylchlorid und wenig unumgesetztes o-Chlortoluol und wenig o-Chlorbenzalchlorid.

Stufe III: Bis noch keine nennenswerten Mengen o-Chlorbenzotrichlorid entstehen. Das Chlorierungsprodukt enthält dann im wesentlichen o-Chlorbenzylchlorid, Benzalchlorid und wenig unumgesetztes o-Chlortoluol.

Stufe IV: Bis praktisch alles o-Chlortoluol umgesetzt ist. Das Chlorierungsprodukt enthält dann im wesent lichen o-Chlorbenzylchlorid, o-Chlorbenzalchlorid und wenig o-Chlorbenzotrichlorid.

Stufe V: Bis die maximale Menge o-Chlorbenzalchlorid entstanden ist. Das Chlorierungsprodukt enthält dann im wesentlichen o-Chlorbenzalchlorid und wenig o-Chlorbenzylchlorid und o-Chlorbenzotrichlorid.

Stufe VI: Bis praktisch kein o-Chlorbenzylchlorid mehr vorhanden ist. Das Chlorierungsprodukt enthält dann im wesentlichen o-Chlorbenzalchlorid und o-Chlorbenzotrichlorid.

Stufe VII: Bis praktisch noch keine durch Überchlorierung und/oder chlorierende Spaltung verursachte Nebenprodukte entstehen. Das Chlorierungsprodukt enthält dann im wesentlichen o-Chlorbenzotrichlorid und wenig o-Chlorbenzalchlorid.

Stufe VIII: Bis praktisch kein o-Chlorbenzalchlorid mehr vorhanden ist Das Chlorierungsprodukt enthält dann im wesentlichen o-Chlorbenzotrichlorid und durch Überchlorierung und/oder chlorierende Spaltung entstandene Nebenprodukte.

Um reines o-Chlorbenzylchlorid, reines o-Chlorbenzalchlorid und/oder reines o-Chlorbenzotrichlorid zu erhalten, wie es für die Weiterverarbeitung notwendig ist, ist also in jedem Falle nach der Chlorierung eine Destillation durchzuführen.

Auch bei der Chlorierung anderer Alkylaromaten als o-Chlortoluol treten entsprechende Chlorierungsproduktmischungen auf, die ebenso durch Destillation aufgearbeitet werden müssen, um zu weiterverwendbaren Produkten zu gelangen.

Unter den Bedingungen für derartige destillative Aufarbeitungen (hohe Temperaturen, vielbödige Kolonnen, hohe Rücklaufverhältnisse; diese müssen wegen der hohen Siedepunkte und der geringen Siedepunktsunterschiede der zu trennenden Substanzen angewendet werden) finden in den Destillationskolonnen und Nebenaggregaten in erheblichem Umfang Nebenreaktionen statt. Bei den Nebenaggregaten kann es sich z.B. um Kondensatoren, Verdampfer, Rohrleitungen, Verteiler, Randabweiser, Probeentnahmestellen, Meßgeräte, Füllkörper und Packungen (z.B. aus keramischem Material) handeln. Bei den Nebenreaktionen kann es sich z.B. um Kondensationsreaktionen zwischen 2 und/oder mehr Molekülen seitenkettenchlorierter Alkylaromaten, spontane Zersetzungen, Polymerisationen und Korrosionen handeln. Dies führt nicht nur zu Verlusten an Produkten und Apparatematerialien, sondern, was noch schwerwiegender ist, auch zu Ablagerungen in den Destillationskolonnen und/oder Nebenaggregaten, und damit zu Verengungen und Verstopfungen in diesen Geräten. Häufig kann man deshalb Destillationsanlagen zur Aufarbeitung von Gemischen, die seitenkettenchlorierte Alkylaromaten enthalten, nur kurze Zeit betreiben, muß sie dann

abstellen, zerlegen und gebildete Ablagerungen entfernen. Das ist sehr arbeits- und kostenintensiv und gestattet keine gleichmäßige Arbeitsweise.

Versuche, diese Probleme durch den Einsatz von Kolonnen mit speziellen Packungen und/oder durch die Anwendung spezieller Materialien (z.B. Emaille, Sondermetalle, Keramik, Glas, Fluor enthaltende Polymere) zu überwinden konnten nicht befriedigen. So neigt beispielsweise Emaille bei den vorliegenden Temperaturbeanspruchungen zum Abplatzen, Sondermetalle (wie Tantal) sind unverhältnismäßig teuer, Meßgeräte sind häufig nicht aus solchen Materialien erhältlich und auch keramische Packungen sind nicht völlig inert.

Es wurde nun ein verbessertes Verfahren zur destillativen Aufarbeitung von Gemischen, die seitenkettenchlorierte Alkylaromaten enthalten, gefunden, das dadurch gekennzeichnet ist, daß man das zu destillierende Gemisch vor der Destillation bei erhöhter Temperatur mit einem inerten Gas ausbläst und die Destillation in Gegenwart von Aminen und/oder chlorierten Aminen durchführt.

In das erfindungsgemäße Verfahren einsetzbare Gemische, die seitenkettenchlorierte Alkylaromaten enthalten, können von verschiedener Art sein. Beispielsweise können sie Alkylaromaten der Formel (I) enthalten,

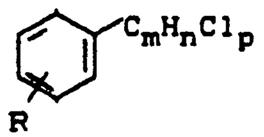

(I),

in der
m für eine ganze Zahl von 1 bis 4,
n für Null oder eine ganze Zahl von 1 bis 2 m und
p für eine ganze Zahl von 1 bis 2 m + 1 stehen,
wobei n + p stets 2 m + 1 ergibt, und
R 1 bis 3 Halogenatome, Alkyl oder Nitro
bedeutet.

Soweit R für 1 bis 3 Halogenatome steht kann es sich beispielsweise um Fluor-, Chlor- und/oder Bromatome handeln. Soweit R für Alkyl steht kann es sich beispielsweise um $C_1$- bis $C_4$-Alkyl handeln. Vorzugsweise bedeutet R 1 bis 2 Chloratome, Methyl, Ethyl oder Nitro.

Einsetzbare Gemische können eine oder mehrere Verbindungen der Formel (I) enthalten. Außerdem können einsetzbare Gemische zusätzlich beispielsweise Verbindungen der Formel (II) enthalten,

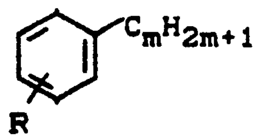

(II),

in der
m und R die bei Formel (I) angegebene Bedeutung haben,
und/oder Verbindungen, die aus Verbindungen der Formeln (I) und/oder (II) durch Kondensation und/oder Zersetzung hervorgegangen sind.

Bevorzugt werden in das erfindungsgemäße Verfahren Gemische eingesetzt, wie sie bei der Seitenkettenchlorierung einer Verbindung der Formel (II) erhalten werden, wobei die Chlorierung beliebig weit durchgeführt worden sein kann. Bei der Chlorierung von o-Chlortoluol kann die Chlorierung beispielsweise bis zu einer der eingangs beschriebenen Stufen I bis VIII oder, bei anderem Einsatzmaterial, bis zu einer entsprechenden Stufe durchgeführt worden seine. Die Chlorierung kann dabei mit einem beliebigen Chlorierungsmittel durchgeführt worden sein Ein bevorzugtes Chlorierungsmittel ist elementares Chlor.

Besonders bevorzugt sind Gemische, die solche Alkylaromaten der Formel (I) enthalten, bei denen R für 1 oder 2 Chloratome und m für 1 steht.

Ganz besonders bevorzugt sind Gemische, die bei der Seitenkettenchlorierung von Chlortoluolen erhalten werden.

In das erfindungsgemäße Verfahren einsetzbare Gemische können beliebige Mengen an seitenkettenchlorierten Alkylaromaten enthalten. Beispielsweise kann die Summe aller vorhandenen seitenkettenchlorierten Alkylaromaten in diesen Gemischen 15 bis 100 Gew.-% betragen. Bevorzugt enthalten diese Gemische 20 bis 99 Gew.-% eines oder mehrerer seitenkettenchlorierter Alkylaromaten.

Das Ausblasen mit einem inerten Gas erfolgt bei erhöhter Temperatur. Geeignete Temperaturen sind beispielsweise solche im Bereich 100 bis 200° C. Bevorzugt wird das Ausblasen bei einer Temperatur von 120 bis 180° C durchgeführt. Als inertes Gas kommt in erster Linie wasserfreier Stickstoff in Frage, aber auch Edelgase oder Gemische von Stickstoff mit Edelgasen. Da Wasserdampf und Sauerstoff unter den anzuwendenden Bedingungen nicht als inert anzusehen sind, sollte das einzusetzende inerte Gas möglichst weitgehend frei von Wasserdampf und Sauerstoff sein.

Wenn man Gemische aus der Seitenkettenchlorierung von Alkylaromaten in das erfindungsgemäße Verfahren einsetzt ist es vorteilhaft, das Ausblasen mit einem inerten Gas im Anschluß an die Chlorierung durchzuführen. Auf diese Weise wird kein Aufwand nötig, um für das Ausblasen geeignete Temperaturen einzustellen, denn die Seitenkettenchlorierung von Alkylaromaten wird normalerweise bei Temperaturen durchgeführt, die auch für das erfindungsgemäß durchzuführende Ausblasen mit einem inerten Gas geeignet sind.

Der Ausblasvorgang kann kontinuierlich oder diskontinuierlich durchgeführt werden und beispielsweise so lange, bis das anschließend zu destillierende Gemisch einen Gehalt an nicht-gebundenem Chlor von <10 ppm und Gehalte an Chlorwasserstoff und Wasser von jeweils <30 ppm aufweist. Dies ist im allgemeinen der Fall, wenn die 1 bis 5-fache Volumenmenge Inertgas, bezogen auf das seitenkettenchlorierte Alkylaromaten enthaltende Einsatzgemisch, zum Ausblasen verwendet wird.

Die Amine und/oder chlorierten Amine sind vorzugsweise in allen Teilen der Destillation zugegen. Man kann deshalb beispielsweise bei einer kontinuierlichen Destillation des seitenkettenchlorierte Alkylaromaten enthaltenden Gemischs so verfahren, daß man Amine und/oder chlorierte Amine mit verschiedenen Siedepunkten hinzufügt, und zwar so, daß mindestens ein Amin und/oder chloriertes Amin über den Kopf und mindestens ein Amin und/oder chloriertes Amin über den Sumpf der jeweiligen Destillierkolonne ausgetragen wird. Die Zugabe der Amine und/oder Chloramine kann gemeinsam oder getrennt an beliebiger Stelle einer kontinuierlich betriebenen Destillationskolonne erfolgen, beispielsweise in der Nähe der Zugabe von oder zusammen mit dem seitenkettenchlorierte Alkylaromaten enthaltenden Gemisch.

Bei einer diskontinuierlichen Destillation kann man beispielsweise so verfahren, daß man in die Blase laufend ein relativ niedrig siedendes Amin und/oder chloriertes Amin zugibt, das über Kopf ausgetragen wird.

Selbstverständlich sind auch andere Zugabestellen und Zugabeformen möglich.

Amine und/oder chlorierte Amine können beispielsweise in einer Gesamtmenge von 0,02 bis 0,2 Gew.-%, bezogen auf das zu destillierende Gemisch, zugegen sind. Da bei der Destillation anfallende Kopfprodukte häufig in die Chlorierung zurückgeführt werden, um letztlich höher chlorierte Produkte herzustellen, und erfindungsgemäß erhaltene Kopfprodukte, Amine und/oder chlorierte Amine enthalten, ist es nicht immer notwendig Amine und/oder chlorierte Amine von außen zuzudosieren. Häufig kann auf die beschriebene Weise über kürzere oder längere Zeit ein Aminkreislauf und/oder ein Kreislauf von chlorierten Aminen aufrechterhalten werden, in den nur von Zeit zu Zeit Nachdosierungen erfolgen müssen.

Als Amine und/oder chlorierte Amine kommen z.B. aliphatische und aromatische Typen in Frage, wobei Einzelverbindungen oder Gemische zum Einsatz gelangen können. Bevorzugt sind Stickstoff enthaltende Heterocyclen und/oder deren kernchlorierte Derivate. Besonders bevorzugt sind Pyridin, Chinolin und kernchlorierte Derivate von diesen, die 1 bis 3 Chloratome enthalten, sowie Gemische solcher Verbindungen.

Die destillative Aufarbeitung von Gemischen, die seitenkettenchlorierte Alkylaromaten enthalten, kann sonst, z.B. in Bezug auf Geräte, Gerätematerialien, Abmessungen, Rücklaufverhältnisse, Temperaturen, Drucke, Verweilzeiten etc., wie üblich durchgeführt werden. Die Rücklaufverhältnisse können beispielsweise im Bereich 1 bis 40, vorzugsweise im Bereich 1 bis 8 liegen, die Temperaturen können beispielsweise zwischen 100 und 250° C, vorzugsweise zwischen 100 und 200° C betragen, der Druck kann beispielsweise zwischen 1 und 500 mbar, vorzugsweise zwischen 30 und 150 mbar sein und die Verweilzeit kann bei kontinuierlicher Arbeitsweise beispielsweise zwischen 10 und 120 Minuten, vorzugsweise zwischen 20 und 40 Minuten und bei diskontinuierlicher Arbeitsweise beispielsweise zwischen 1 und 100 Stunden, vorzugsweise zwischen 2 und 10 Stunden liegen.

Das erfindungsgemäße Verfahren führt zu drastischen Erniedrigungen der Korrosion und der Nebenreaktionen während der Destillation von Gemischen, die seitenkettenchlorierte Alkylaromaten enthalten. Damit können auf die erfindungsgemäße Weise derartige Destillationen lange Zeiten störungsfrei durchgeführt werden. So ist es möglich aus den eingesetzten Gemischen höhere Anteile einzelner seitenkettenchlorierter Alkylaromaten und/oder einzelne seitenkettenchlorierte Alkylaromaten in höherer Reinheit zu isolieren, da ein höherer Destillationsaufwand (z.B. höhere Kolonnen, mehrfache Destillationen) nicht mehr mit einem höheren Risiko von Verstopfungen und Produktverunreinigungen verbunden ist.

Beispiel 1

Verschiedene seitenkettenchlorierte Alkylaromaten mit und ohne Zusätze wurden hinsichtlich ihres thermischen Zersetzungsverhaltens mittels Differenzialthermoanalyse (Gerät Mettler TA 2000, Übersichtsmessung mit kontinuierlicher Temperaturerhöhung um 5 K pro Minute) untersucht. Die Ergebnisse sind im Detail in Tabelle 1 zusammengefaßt, worin jeweils der Temperaturbereich angegeben ist, in dem exotherme Zersetzungsreaktionen beobachtet wurden.

Es ist zu ersehen, daß seitenkettenchlorierte Alkylaromaten in reiner Form ("ohne Zusatz") thermisch bis mindestens 250° C stabil sind. Nach Zusätzen geringer Mengen an Rost, Eisen(III)-chlorid oder elementarem Eisen, die bei der Handhabung derartiger Substanzen in aus Metall hergestellten Apparaten herrschende Verhältnisse simulieren, sind die seitenkettenchlorierten Alkylaromaten signifikant weniger thermisch stabil. Bei Rostzusatz treten bereits ab ca. 70° C, bei Eisen(III)-chlorid-Zusatz bereits ab 35 bis 80° C und beim Zusatz von elementarem Eisen ab 160 bis 180° C exotherme Zersetzungen auf. Wird außerdem gemäß der vorliegenden Erfindung ein Amin zugegeben (hier Pyridin), so steigt in den Rost und Eisen(III)-chlorid enthaltenden Proben die thermische Stabilität auf ca. 250 bis 300° C an und in den elementares Eisen enthaltenden Proben auf ca. 230 bis 250° C.

Tabelle 1

| seitenkettenchlorierter Alkylaromat | ohne Zusatz | Temperaturbereiche exothermer Zersetzungsreaktionen mit Zusatz von | | | | | |
|---|---|---|---|---|---|---|---|
| | | 0,1 Gew.-% Rost | 0,1 Gew.-% Rost und 0,1 Gew.-% Pyridin | 0,16 Gew.-% $FeCl_3$ | 0,09 Gew.-% $FeCl_3$ und 0,1 Gew.-% Pyridin | 0,1 Gew.-% elementares Eisen | 0,1 Gew.-% elementares Eisen und 0,1 Gew.-% Pyridin |
| a) o-Chlorbenzylchlorid | über 300° C | 80-160° C | über 300° C | 80-165° C | über 300° C | 170-190° C | über 230° C |
| b) o-Chlorbenzalchlorid | über 300° C | 85-145° C | über 300° C | unter 200° C | über 300° C | 120-215° C | über 230° C |
| c) o-Chlorbenzotrichlorid | über 300° C | 175-280° C | über 280° C | 25° C | über 300° C | 160-270° C | über 250° C |
| d) 2,4-Dichlorbenzylchlorid | über 250° C | 90-120° C | über 290° C | 35-60° C | über 280° C | 160-250° C | über 260° C |
| e) 2,4-Dichlorbenzalchlorid | über 330° C | ab 70° C | über 250° C | 80-200° C | über 260° C | ab 180° C | über 250° C |

Beispiele 2 bis 8

In entsprechender Weise wie in Beispiel 1 wurde o-Chlorbenzylchlorid ohne Zusätze, mit Zusatz von Eisen(III)-chlorid und mit Zusätzen von Eisen(III)-chlorid und verschiedenen Aminen und Chloraminen hinsichtlich des thermischen Zersetzungsverhaltens mittels Differenzialthermoanalyse untersucht. Die Ergebnisse sind im Detail in Tabelle 2 zusammengefaßt, worin jeweils die Temperatur angegeben ist, bei der eine exotherme Zersetzungsreaktion begann.

Es ist zu ersehen, daß reines o-Chlorbenzylchlorid bis 300° C stabil ist. Nach Zusatz von Eisen(III)-chlorid, was die bei der Handhabung von o-Chlorbenzylchlorid in aus Metall hergestellten Apparaten herrschenden Verhältnisse simuliert, ist o-Chlorbenzylchlorid weit weniger thermisch stabil, da dann Zersetzungsreaktionen bereits bei 95° C beginnen. Werden entsprechend der vorliegenden Erfindung (siehe Beispiele 4 bis 8) Amine oder Chloramine zugefügt, so steigt die thermische Stabilität deutlich an. Zersetzungsreaktionen beginnen dann erst bei Temperaturen von 135 bis 280° C.

Tabelle 2

| Beispiel Nr. | untersuchte Substanz | Beginn thermischer Zersetzungsreaktionen |
|---|---|---|
| 2 | o-Chlorbenzylchlorid ohne Zusätze | 300° C |
| 3 | o-Chlorbenzylchlorid + 200 ppm $FeCl_3$ | 95° C |
| 4 | o-Chlorbenzylchlorid + 200 ppm $FeCl_3$ + 1000 ppm Pyridin | 250° C |
| 5 | o-Chlorbenzylchlorid + 200 ppm $FeCl_3$ + 1000 ppm 2,6-Dichlorpyrimidin | 135° C |
| 6 | o-Chlorbenzylchlorid + 200 ppm $FeCl_3$ + 1000 ppm 2,3,5-Trichlorpyrimidin | 140° C |
| 7 | o-Chlorbenzylchlorid + 200 ppm $FeCl_3$ + 1000 ppm 3-Chlorpyrimidin | 280° C |
| 8 | o-Chlorbenzylchlorid + 200 ppm $FeCl_3$ + 1000 ppm Chinolin | 190° C |

Beispiel 9

Aus o-Chlortoluol, das in einem Stahlgefäß gelagert worden war, und rückgeführtem Kopfprodukt (siehe unten) wurde durch eine übliche kontinuierliche Chlorierung in einer emaillierten Apparatur ein Gemisch erhalten, das 0,5 Gew.-% o-Chlortoluol, 57,0 Gew.-% o-Chlorbenzylchlorid, 42,0 Gew.-% o-Chlorbenzalchlorid und 0,5 Gew.-% o-Chlorbenzotrichlorid enthielt. 1 kg dieses Gemisches wurde diskontinuierilch in einer emaillierten Apparatur bei einer Temperatur von 150° C im Verlauf von 4 Stunden mit 10 l trockenem Stickstoff ausgeblasen. Danach wurde in dem Gemisch der Gehalt an Aminen (= Pyridin + kernchlorierte Pyrimidine) kontrolliert und gegebenenfalls durch Zugabe von Pyridin auf einen Wert von 0,02 ± 0,001 Gew.-% eingestellt. Anschließend wurde das Gemisch in einer Kolonne aus Nickel (Länge 4,5 m, Innendurchmesser 70 mm, bis zu einer Höhe von 4 m gefüllt mit Kerapak®-Füllkörpern) kontinuierlich destilliert. Die Sumpftemperatur betrug 163° C, die Kopftemperatur 138° C, der Druck im Sumpf 130 mbar, der Druck am Kopf 100 mbar und das Rücklaufverhältnis (Rücklauf zur Entnahme am Kopf) 1,5:1. Pro Stunde wurden 0,58 kg eines Kopfproduktes abgetrennt, das 0,8 Gew.-% o-Chlortoluol, 98 Gew.-% o-Chlorbenzoylchlorid, 1,2 Gew.-% o-Chlorbenzalchlorid und geringe Mengen Pyridin und kernchlorierte Pyridine enthielt und in die Chlorierung zurückgeführt wurde, sowie 0,420 kg eines Sumpfproduktes, das 0,3 Gew.-% o-Chlorbenzylchlorid, 98,5 Gew.-% o-Chlorbenzalchlorid, 1,2 Gew.-% o-Chlorbenzotrichlorid und geringe Mengen Pyridin und kernchlorierte Pyridine enthielt. Diese Destillation wurde über 100 Stunden lang durchgeführt, ohne daß Zersetzungen beobachtet wurden

Beispiel 10

Es wurde verfahren wie in Beispiel 9, jedoch lag nach der Chlorierung folgendes Gemisch vor:
1,0 Gew.-% o-Chlorbenzylchlorid,
85,0 Gew.-% o-Chlorbenzalchlorid,
14,0 Gew.-% o-Chlorbenzotrichlorid.
2,3 kg dieses Gemisches wurden diskontinuierlich bei 150° C im Verlauf von 4 Stunden mit 23 l trockenem Stickstoff ausgeblasen, dann der Amingehalt wie bei Beispiel 9 angegeben eingestellt. Bei der Destillation betrug die Sumpftemperatur 165° C, die Kopftemperatur 146° C, der Druck im Sumpf 130 mbar, der Druck am Kopf 110 mbar und das Rücklaufverhältnis 30:1. Pro -Stunde wurden 0,033 kg Kopfprodukt enthaltend 60 Gew.-% o-Chlorbenzylchlorid, 40 Gew.-% o-Chlorbenzalchlorid und geringe Mengen Pyridin und kernchlorierte Pyridine und 2,297 kg Sumpfprodukt enthaltend 0,1 Gew.-% o-Chlorbenzylchlorid, 85,6 Gew.-% o-Chlorbenzalchlorid, 14,3 Gew.-% o-Chlorbenzotrichlorid und geringe Mengen Pyridin und kernchlorierte Pyridine abgetrennt. Diese Destillation wurde über 100 Stunden lang durchgeführt, ohne das Zersetzungen beobachtet wurden.

Beispiel 11

Es wurde verfahren wie in Beispiel 9, jedoch wurde p-Chlortoluol chloriert, wobei nach der Chlorierung ein Gemisch folgender Zusammensetzung vorlag:
0,1 Gew.-% p-Chlortoluol,
33,3 Gew.-% p-Chlorbenzylchlorid,
60,0 Gew.-% p-Chlorbenzalchlorid und
6,6 Gew.-% p-Chlorbenzotrichlorid.
0,975 kg dieses Gemisches wurden diskontinuierlich bei 160° C im Verlauf von 4 Stunden mit 10 l trockenem Stickstoff ausgeblasen, dann der Amingehalt wie in Beispiel 9 beschrieben eingestellt. Bei der Destillation betrug die Sumpftemperatur 167° C, die Kopftemperatur 144° C, der Druck im Sumpf 130 mbar, der Druck am Kopf 100 mbar und das Rücklaufverhältnis 2:1. Pro Stunde wurden 0,326 kg Kopfprodukt enthaltend 0,3 Gew.-% p-Chlortoluol, 99 Gew.-% p-Chlorbenzylchlorid, 0,7 Gew.-% p-Chlorbenzalchlorid und geringe Mengen Pyridin und kernchlorierte Pyridine und 0,649 kg Sumpfprodukt enthaltend 0,3 Gew.-% p-Chlorbenzylchlorid, 89,7 Gew.-% p-Chlorbenzalchlorid, 10 Gew.-% p-Chlorbenzotrichlorid und geringe Mengen Pyridin und kernchlorierte Pyridine abgetrennt. Diese Destillation wurde über 100 Stunden lang durchgeführt, ohne das Zersetzungen beobachtet wurden.

Beispiel 12

Es wurde verfahren wie in Beispiel 9, jedoch wurde 2,4-Dichlortoluol chloriert, wobei nach der Chlorierung ein Gemisch folgender Zusammensetzung vorlag:
20 Gew.-% 2,4-Dichlorbenzalchlorid und
80 Gew.-% 2,4-Dichlorbenzotrichlorid.
1,68 kg dieses Gemisches wurden diskontinuierlich bei 160° C im Verlauf von 4 Stunden mit 17 l trockenem Stickstoff ausgeblasen, dann der Amingehalt wie im Beispiel 9 beschrieben eingestellt. Bei der Destillation betrug die Sumpftemperatur 198° C, die Kopftemperatur 165° C, der Druck im Sumpf 198 mbar, der Druck am Kopf 165 mbar und das Rücklaufverhältnis 1,5:1. Pro Stunde wurden 0,336 kg Kopfprodukt enthaltend 99,2 Gew.-% 2,4-Dichlorbenzalchlorid, 0,8 Gew.-% 2,4-Dichlorbenzotrichlorid und geringe Mengen Pyridin und kernchlorierte Pyridine und 1,344 kg Sumpfprodukt enthaltend 0,2 Gew.-% 2,4-Dichlorbenzalchlorid, 99,8 Gew.-% 2,4-Dichlorbenzotrichlorid und geringe Mengen Pyridin und kernchlorierte Pyridine abgetrennt. Diese Destillation wurde über 100 Stunden lang durchgeführt, ohne das Zersetzungen beobachtet wurden.

Beispiel 13 (zum Vergleich)

9 kg eines Gemisches aus 23 Gew.-% 2,4- und 2,5-Dichlorbenzalchlorid, 35 Gew.-% 2,6-Dichlorbenzalchlorid und 42 Gew.-% 2,4- und 2,5-Dichlorbenzotrichlorid, das analog Beispiel 9 aus einem Dichlortoluolgemisch hergestellt worden war, wurden mit 30 l trockenem Stickstoff bei 160° C im Verlauf von 4 Stunden ausgeblasen. In Abwesenheit von Pyridin und kernchlorierten Pyridinen wurde das ausgeblasene Gemisch in einer Apparatur wie in Beispiel 9 beschrieben destilliert. Nachdem bei einer Sumpftemperatur von 190° C, einer Kopftemperatur von 170° C, einem Druck im Sumpf von 100 mbar, einem Druck am Kopf von 70 mbar und einem Rücklaufverhältnis von 3:1 2 kg abdestilliert worden waren bildeten die im Sumpf verbliebenen 7 kg ein verharztes Produkt, das nicht mehr destillierbar war.

## Ansprüche

1. Verfahren zur destillativen Aufarbeitung von Gemischen, die seitenkettenchlorierte Alkylaromaten enthalten, dadurch gekennzeichnet, daß man das zu destillierende Gemisch vor der Destillation bei erhöhter Temperatur mit einem inerten Gas ausbläst und die Destillation in Gegenwart von Aminen und/oder chlorierten Aminen durchführt.

2. Verfahren nach Anspruch 1, dadurch gekennzeichnet, daß man Gemische einsetzt, die Alkylaromaten der Formel (I) enthalten,

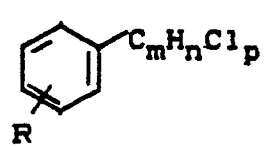 (I),

in der
m für eine ganze Zahl von 1 bis 4,
n für Null oder eine ganze Zahl von 1 bis 2 m und
p für eine ganze Zahl von 1 bis 2 m + 1 stehen,
wobei n + p stets 2 m + 1 ergibt, und
R 1 bis 3 Halogenatome, Alkyl oder Nitro
bedeutet.

3. Verfahren nach Anspruch 2, dadurch gekennzeichnet, daß in Formel (I) R für 1 bis 2 Chloratome, Methyl, Ethyl oder Nitro steht.

4. Verfahren nach Ansprüchen 1 bis 3, dadurch gekennzeichnet, daß man Gemische einsetzt wie sie bei der Seitenkettenchlorierung einer Verbindung der Formel (II)

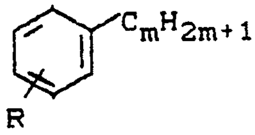

(II),

in der
m für eine ganze Zahl von 1 bis 4 und
R für 1 bis 3 Halogenatome, Alkyl oder Nitro
stehen mit elementarem Chlor erhalten werden.

5. Verfahren nach Anspruch 4, dadurch gekennzeichnet, daß man Gemische einsetzt, die bei der Seitenkettenchlorierung von Chlortoluolen erhalten werden.

6. Verfahren nach Ansprüchen 1 bis 5, dadurch gekennzeichnet, daß man das Ausblasen bei 100 bis 200 °C vornimmt.

7. Verfahren nach Ansprüchen 1 bis 6, dadurch gekennzeichnet, daß man das Ausblasen vornimmt, bis in dem zu destillierenden Gemisch ein Gehalt von nicht-gebundenem Chlor von weniger als 10 ppm und Gehalte von Chlorwasserstoff und Wasser von jeweils weniger als 30 ppm vorliegen.

8. Verfahren nach Ansprüchen 1 bis 7, dadurch gekennzeichnet, daß die Amine und/oder chlorierten Amine in allen Teilen der Destillation zugegen sind,

9. Verfahren nach Ansprüchen 1 bis 8, dadurch gekennzeichnet, daß Amine und/oder Chloramine in einer Gesamtmenge von 0,02 bis 0,2 Gew.-%, bezogen auf das zu destillierende Gemisch, zugegen sind.

10. Verfahren nach Ansprüchen 1 bis 9, dadurch gekennzeichnet, daß als Amine und/oder chlorierte Amine Stickstoff enthaltende Heterocyclen und/oder deren kernchlorierte Derivate zugegen sind.

Europäisches Patentamt

# EUROPÄISCHER RECHERCHENBERICHT

Nummer der Anmeldung

EP 89 12 0982

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DD-A- 121 776 (H. HAACK et al.) * Seite 2, linke Spalte, Zeile 41 - Seite 3, linke Spalte, Zeile 5 * | 1-3,6, 10 | C 07 C 17/38<br>C 07 C 25/02 |
| Y | US-A-3 919 054 (C.H.G. HANDS) * Spalte 1, Zeilen 1-28 * | 1-3,6, 10 | |
| A | DE-B-2 543 992 (DYNAMIT NOBEL AG) * Anspruch 1 * | 1 | |
| A | US-A-2 025 024 (E.C. BRITTON) * Seite 1, rechte Spalte, Zeilen 21-30 * | 1 | |
| A | US-A-2 543 575 (C.C. HARVEY et al.) * Spalte 1, Zeile 47 - Spalte 2, Zeile 34 * | 1 | |
| P,A | EP-A-0 334 025 (TORAY INDUSTRIES, INC.) * Seite 5, Beispiel 2 * | 1 | |

| RECHERCHIERTE SACHGEBIETE (Int. Cl.5) |
|---|
| C 07 C 17/00 |

Der vorliegende Recherchenbericht wurde für alle Patentansprüche erstellt

| Recherchenort | Abschlußdatum der Recherche | Prüfer |
|---|---|---|
| BERLIN | 13-03-1990 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument